# EUROPEAN PATENT APPLICATION

(11) **EP 1 857 819 A1**
(43) Date of publication of application: **21.11.2007**
(21) Application number: 06714554.0
(22) Date of filing: 24.02.2006
(51) Int. Cl.: G01N 33/68, G01N 33/53

(54) **METHOD OF MEASURING EXPOSURE DOSAGE OF IONIZING RADIATION**

(30) Priority: 25.02.2005 JP 2005050497
(71) Applicant: Hiroshima University, Higashihiroshima-shi Hiroshima, 739-8511 (JP); Two Cells Co., Ltd, Hiroshima-shi, Hiroshima 732-0811 (JP)
(72) Inventor: TATSUKA, Masaaki, -shi Hiroshima, 7320066 (JP)
(74) Representative: Forstmeyer, Dietmar
(86) International application number: PCT/JP2006/303415
(87) International publication number: WO 2006/090836

(57) **Abstract**

A method of measuring an exposed dose of ionizing radiation, containing: (a) extracting proteins from a tissue or blood collected from a living body, and (b) determining the content of at least one of products of LyGDI protein degraded by caspase-1 and caspase-3 in the extracted proteins.

## Description

### TECHNICAL FIELD

The present invention relates to a method of measuring an exposed dose of ionizing radiation, wherein a biological influence of exposure ionizing radiations or electromagnetic waves on a living body can be directly known by utilizing biological chemical reaction.

### BACKGROUND ART

For measurement of an exposed dose of ionizing radiation, a physical reaction caused by the ionizing radiation is generally used, and specifically a film badge, a thermoluminescence dosimeter, a pocket dosimeter, and the like are utilized (Actual Radiation Control for Chief, revised 2nd edition, p. 140, 1992, Japan Radioisotope Association).

The film badge comprises a case accommodating a resin film coated with a photographic emulsion, wherein the photographic emulsion upon exposure to an ionizing radiation exhibits, together with a photosensitive action, a blackening action in proportion to the dose of the radiation, and this photosensitive action is utilized to determine the exposed dose of the ionizing radiation. On the other hand, the thermoluminescence dosimeter is a dosimeter utilizing luminescence (fluorescence) caused upon heating of a crystalline material having irradiated with a radiation, wherein an electron and an electron hole are separated from each other in the crystal upon irradiation with an electron radiation, and when recombined with each other by thermal stimulation, they produce luminescence; by this luminescence principle, the exposed dose of the ionizing radiation is determined. Further, the pocket dosimeter is a meter wherein a gas encapsulated in an ionization chamber is ionized depending on the dose of ionizing radiation applied thereto, and from electricity caused upon the ionization, the exposed dose of the ionizing radiation is determined.

These physical measurement methods have high reliability and stability and are handled inexpensively and easily by anyone, but suffer from the following problems:

First, in the physical measurement methods described above, the dose of ionizing radiation applied is measured as the dose of radiation absorbed into the various measuring instruments. However, whether this dose agrees with the dose of radiation absorbed into a living body to directly influence the health of the living body, is questionable, particularly in a low-dose range.

In addition, in connection with this problem, the physical measurement methods described above are those for measuring only the dose of radiation applied to the surface of a living body, and can thus not measure the dose of irradiation having reached the inside of a living body. For example, the film badge responds to exposure to low-permeable ³²P gamma rays, but a majority of such applied irradiations will be reduced by shielding materials such as clothing. Accordingly, the exposed dose measured by the physical methods described above does not always reflect the dose of radiations to which a living body was actually exposed.

Further, the physical measurement methods could not accurately reflect the influence, on a living body, of radiations different in RBE (radiation biological effectiveness). For 1 Gy of neutron radiation and 1 Gy of X-ray, for example, it is known that although the same dose of 1 Gy are assigned to the two, the biological effectiveness of the former is 2 to 10 times as high as that of the latter. In the physical methods described above, however, the two are shown to have the same dose of 1 Gy.

As a matter of course, a concept "effective radiation dose" exists in the physical methods and is legally adopted. For calibrating "effective radiation dose", however, radiation quality and a tissue exposed to radiation should be identified prior to calculation of the exposed dose of radiation. For examining the influence of radiation on one person, for example, the exposed dose should be expressed as the sum of exposed doses for the respective tissues by determining an exposed dose for each tissue according to "tissue weighting factor" for the organ. However, there are subtle individual differences in the weight of each organ and also in "tissue weighting factor", and thus whether the effective radiation dose thus determined accurately reflects the influence of radiation on each person is questionable.

In addition, the physical measurement methods cannot be used in measurement without previously providing a subject with a measuring device such as a film badge. Accordingly, a dose in the case where a civilian was accidentally exposed to radiations, for example, in an atomic-power accident, cannot be directly measured and is thus inevitably indirectly estimated.

In the physical measurement methods, therefore, the influence of ionizing radiation exposed on a living body should be estimated indirectly and unreliably by calculation, and when there is a possibility of exposure, a subject should be provided previously with a measuring device such as a film badge.
Other and further features and advantages of the invention will appear more fully from the following description, appropriately referring to the accompanying drawings.

### DISCLOSURE OF INVENTION

According to the present invention, there is provided the following means:
(1) A method of measuring an exposed dose of ionizing radiation, comprising the steps of:
   (a) extracting proteins from a tissue or blood collected from a living body, and
   (b) determining the content of at least one of products of LyGDI protein degraded by caspase-1 and caspase-3 in the extracted proteins;
(2) The method of measuring an exposed dose of ionizing radiation according to the above item (1), wherein the content of products of LyGDI protein degraded by caspase-1 and products of LyGDI protein degraded by caspase-3 are determined;
(3) The method of measuring an exposed dose of ionizing radiation according to the above item (1) or (2), wherein the content of products of LyGDI protein degraded by caspase-1 and caspase-3 is determined by an immunoblotting method;
(4) The method of measuring an exposed dose of ionizing radiation according to any one of the above items (1) to (3), wherein the protein is extracted from collected thymus, bonemarrow, spleen, intestinal epithelium tissue or blood;
(5) The method of measuring an exposed dose of ionizing radiation according to any one of the above items (1) to (3), wherein the protein is extracted from collected thymus;
(6) The method of measuring an exposed dose of ionizing radiation according to any one of the above items (1) to (3), wherein the protein is extracted from collected blood; and
(7) A kit for measuring an exposed dose of ionizing radiation, which comprises an antibody for determining, by an immunoblotting method, the content of at least one of products of LyGDI protein degraded by caspase-1 and caspase-3 in proteins extracted from a tissue or blood collected from a living body.

One of influences of ionizing radiation on a living body is apoptosis, and it has been observed from a long time ago that apoptosis is caused by irradiation with ionizing radiations in thymus and other tissues. It is known that the function of a tumor suppressor gene product p53 is involved in this kind of apoptosis, and also that a mitochondria-mediated signal system, the activation of its downstream factor i.e. a protease called by caspase group, and its accompanying DNA degradative enzyme are involved in induction of apoptosis, and fragmentation and activation of intramolecular various molecules are caused by apoptosis.

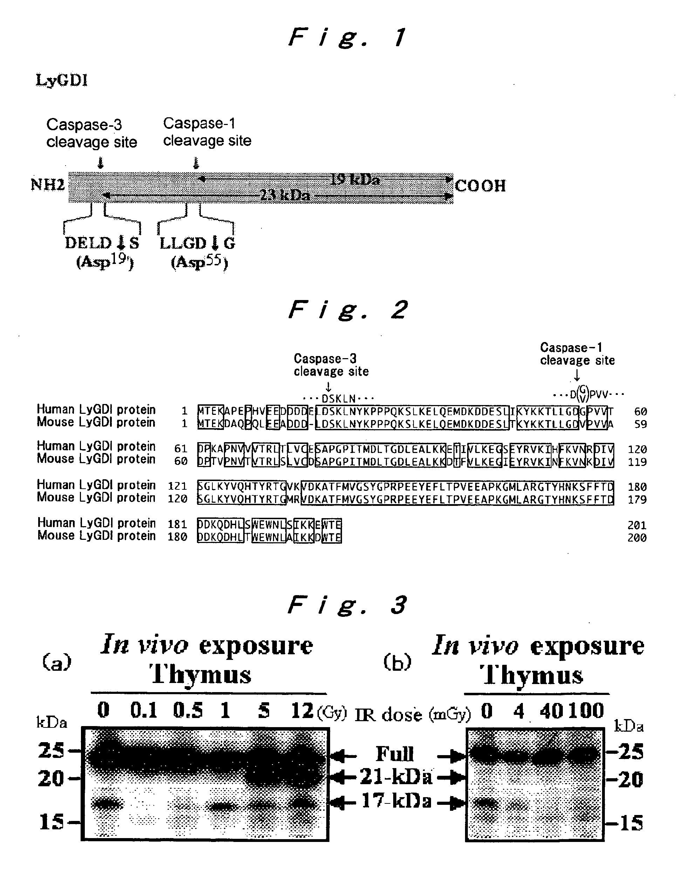

In this connection, as one of proteins cleaved by the group of the above caspase, there is known a LyGDI protein (factor which is a molecule also called GDI-D4, RhoGDI2 or RhoGDIβ and inhibits an activation process by releasing GDP from G protein of Rho family) expressed at high level in blood cells. It is known that human LyGDI protein represented by SEQ ID NO: 1 has a caspase-3 cleavage site at the peptide bond of the C-terminal side of the aspartic acid at the position 19 (or mouse LyGDI protein represented by SEQ ID NO: 2 has it at the position 18) and a caspase-1 cleavage site at the peptide bond of the C-terminal side of the aspartic acid at the position 55 (or mouse LyGDI protein has it at the position 54), as shown in Fig. 1 and Fig. 2.

The present inventor extensively studied the relationship between the LyGDI protein and ionizing radiation, and, as a result, they found that caspase-1 and caspase-3 are activated by irradiation with ionizing radiation (see Radiation Research, 162, pp. 287-295, 2004; Molecular Carcinogenesis, 39, pp. 206-220, 2004; and the like).
The present inventor continued further investigation on the basis of the above study, and, as a result, they found that the content of the products of LyGDI protein degraded by caspase-1 and caspase-3 show a characteristic change depending on the irradiation dose of ionizing radiation, and also that their degraded patterns are varied depending on tissues and blood. On the basis of these findings, the present invention was completed.

According to the present invention, the exposed dose of radiation can be measured on the basis of a change of the structure of the protein collected from a living body.
Specifically, the influence of ionizing radiation on a living body can be directly measured without having a measuring device in advance, by the method of measuring an exposed dose of ionizing radiation according to the present invention. In addition, a film badge or the like is not necessary at the time of exposure, so even in the unlikely event that a living body is exposed to ionizing radiation, such as in an accident in nuclear fuel fabrication, in an accident in an atomic power plant or by accidental leakage of a nuclear material from a nuclear weapon or the like, the influence of the ionizing radiation on the living body can be known more accurately.
In addition, according to the present invention, an exposed dose of ionizing radiation can be measured more accurately than by conventional methods of measuring an exposed dose based on the number or deformation of white blood cells. Further, according to the present invention, a very low exposed dose, which cannot be grasped from a change in white blood cells, can also be measured by a blood examination.

### BRIEF DESCRIPTION OF DRAWINGS

Fig. 1 is a schematic illustration showing the structure of human LyGDI protein.
Fig. 2 is a schematic illustration showing amino acid sequences of human LyGDI protein and mouse LyGDI protein and caspase-1 and caspase-3 cleavage sites.
Fig. 3 shows a result of immunoblotting of proteins extracted from mouse thymus.
Fig. 4 schematically shows the dose of irradiation to which mouse thymus was exposed, and changes in the content of the products degraded by caspase-1 and caspase-3.
Fig. 5 shows a result of immunoblotting of proteins extracted from mouse bonemarrow.
Fig. 6 shows a result of immunoblotting of proteins extracted from mouse spleen.
Fig. 7 shows a result of immunoblotting of proteins extracted from mouse intestinal epithelium tissue.
Fig. 8 shows a result of immunoblotting of proteins extracted from mouse blood.
Fig. 9 shows a result of immunoblotting of proteins extracted from white blood cells in human peripheral blood.
Fig. 10 shows a result of immunoblotting of proteins extracted from white blood cells in human peripheral blood.

### BEST MODE FOR CARRYING OUT THE INVENTION

When a living body is exposed to ionizing radiation, patterns of products of LyGDI degraded by caspase-1 and caspase-3 changes depending on the exposed dose. Based on this finding, the present invention is characterized by measuring the dose of ionizing radiation to which a living body was exposed.

Specifically, the method of measuring an exposed dose of ionizing radiation according to the present invention comprises the steps of: (a) extracting proteins from a tissue or blood collected from a living body, and (b) determining the content of at least one of products of LyGDI protein degraded by caspase-1 and caspase-3 in the extracted proteins. Hereinafter, related subjects are described in detail.

### (Object to be measured)

An object to be measured for the exposed dose of ionizing radiation by this method is not particularly limited insofar as the object is a tissue or blood expressing LyGDI protein constitutively. However, taking easy occurrence of the caspase decomposition reaction into consideration, the object is preferably thymus, bonemarrow, spleen, intestinal epithelium tissue, or blood, among which the thymus is preferable, and blood can also be satisfactorily used for the measuring. The blood includes peripheral blood containing thymus-derived cells (T cells). In addition, a living body from which such tissue or blood is to be collected includes a living body of a mammal such as mouse, dog, cat, swine or bovine, and blood may be collected from humans, and the tissue may be collected from a human dead body. Of the blood, peripheral blood can be easily collected. Thus, the peripheral blood is preferably used as the blood to be used for measuring an exposed dose of ionizing radiation according to the present invention.

### (Collection of tissue or blood and extraction of protein)

Collection of a tissue or blood from a living body can be carried out by a usual method; specifically, the tissue can be collected by a surgical method using a surgical knife, while blood can be collected by suction into a syringe and blood cells are subsequently separated by centrifugation. Extraction of the protein can also be carried out by a usual method, specifically by suspending a tissue, or blood cells, in a buffer and disrupting the tissue or cells with a homogenizer or a French press. In this connection, if necessary, a denaturant, an antioxidant or the like may be added to the buffer; and a nucleic acid such as DNA in a disrupted cellular dispersion, and fat in a cell membrane, may be removed.

### (Determination method)

The method of determining content of the products degraded by caspase-1 and caspase-3 (which are also referred to as Δ55-LyGDI and Δ19-LyGDI, respectively, in this specification) can be used without any particular limitation insofar as the content of a certain protein can be specifically determined, and particularly a method by using antigen/antibody reaction, specifically an immunoblotting method (Western blotting method) and an ELISA method, can be used. As the antibody, an anti-LyGDI antibody to the LyGDI protein and antibodies to the products degraded by caspase-1 and caspase-3 can be used.

Now, the LyGDI protein and the products degraded by caspase are described.
By way of example, human- and mouse-derived LyGDI proteins are described. Amino acid sequences of these proteins are shown in Fig. 2 and represented by SEQ ID NOS: 1 and 2.
The caspase-3 cleavage site in the LyGDI protein is the peptide bond of the C-terminal side of the aspartic acid at the position 19 in the case of human or the peptide bond of the C-terminal side of the aspartic acid at the position 18 in the case of mouse. On the other hand, the caspase-1 cleavage site in the LyGDI protein is the peptide bond of the C-terminal side of the aspartic acid at the position 55 in the case of human or the peptide bond of the C-terminal side of the aspartic acid at the position 54 in the case of mouse.
Accordingly, the amino acid sequence of products of the human LyGDI protein degraded by caspase-3 is represented by SEQ ID NO: 3; the amino acid sequence of products of the mouse LyGDI protein degraded by caspase-3 is represented by SEQ ID NO: 4; the amino acid sequence of products of the human LyGDI protein degraded by caspase-1 is represented by SEQ ID NO: 5; and the amino acid sequence of products of the mouse LyGDI protein degraded by caspase-1 is represented by SEQ ID NO: 6.
Herein, in this specification, the products degraded by caspase-1 and caspase-3 include proteins having amino acid sequences which are represented by SEQ ID NOS: 3 to 6, wherein one or several amino acids are deleted, substituted and/or added in the SEQ ID NOS: 3 to 6, and said sequences are degraded by caspase-1 or caspase-3.

When the content of the products degraded by caspase-1 or caspase-3 is determined by an immunoblotting method, the immunoblotting method known in the art can be used. Specifically, proteins are extracted with a buffer solution containing a denaturant such as sodium dodecyl sulfate (SDS) and then subjected to a polyacrylamide gel electrophoresis. After the electrophoresis is competed, the proteins on gel are transferred onto a PVDF membrane, a nitrocellulose membrane or the like and then visualized with an anti-LyGDI protein antibody or the like thereby determining the content of the products degraded by caspase-1 or caspase-3.

When the content of the products degraded by caspase-1 or caspase-3 is to be determined by an ELISA method, the ELISA method known in the art can be used. Specifically, an anti-LyGDI protein antibody or the like is bound to a solid phase of polystyrene or the like, and a protein extract solution is added thereto and then visualized by reaction with an enzyme-labeled antibody thereby determining the content of the products degraded by caspase-1 or caspase-3 bound to the solid phase.

In this connection, from the viewpoint of degradation of LyGDI protein, the biological reaction which depends on the dose at the exposure to ionizing radiation, is classified into the following two phases.
One phase is a phase observed in a dose range of 1 Gy or more, accompanying activation of caspase-3 with an intracellular apoptosis-inducing signal turning ON to give the Δ19-LyGDI. The other phase is a phase observed in a lower dose range wherein it cannot be detected that the apoptosis-inducing signal is turning ON, and instead, the Δ55-LyGDI is not found.
As described above, the pattern of the products degraded by caspase-1 is different from that of the products degraded by caspase-3, so that upon exposure to a very low dose of ionizing radiation, two indicators, that is, the content of the products of the LyGDI protein degraded by caspase-1 and the content of the products of the LyGDI protein degraded by caspase-3 are preferably determined in order to measure the exposed dose accurately.

As shown in Examples 1-4 and 1-5, there are cases where either the products degraded by caspase-1 and caspase-3 do not exist depending on types of tissues and blood. In addition, as shown in Example 1-4, there is the case where as the exposed dose of ionizing radiation is increased, a decrease in the content of the full-length LyGDI and the Δ19-LyGDI is observed without increasing the content of the Δ19-LyGDI in the extracted proteins.
For measuring the exposed dose of ionizing radiation more accurately, therefore, it is preferable to determine the content of the full-length LyGDI, depending on the case, in addition to the content of the products degraded by caspase-1 or caspase-3 in the extracted proteins.
Herein, in this specification, the full-length LyGDI includes, for example, a protein comprising the amino acid sequence represented by SEQ ID NO: 1 or 2, wherein one or several amino acids deleted, substituted and/or added in SEQ ID NO: 1 or 2, and said sequence contains a caspase-1 or caspase-3 cleavage site.

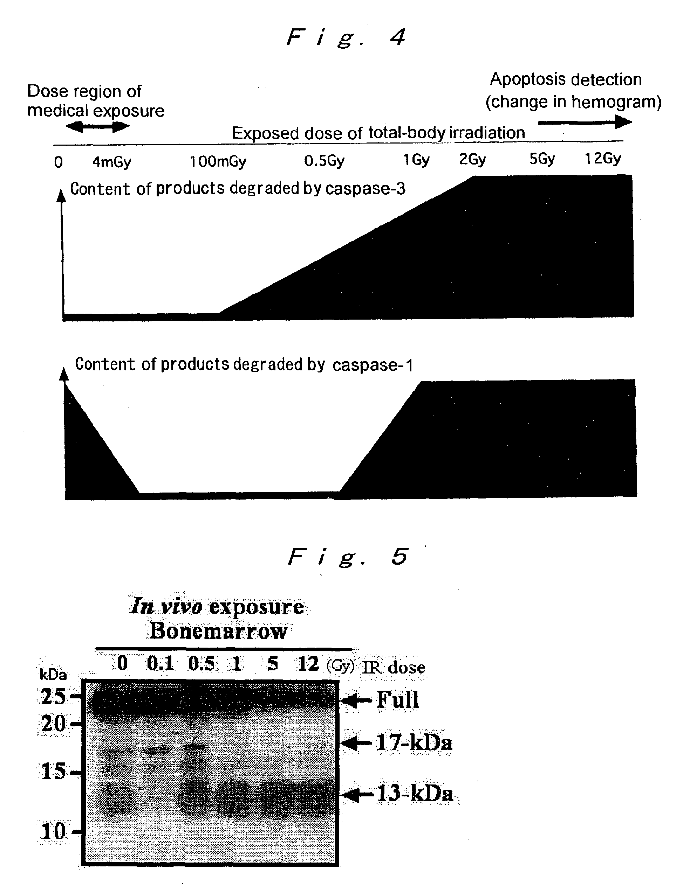

In the present invention, an exposed dose of ionizing radiation can be determined according to the following method.
First, a schematic illustration showing a change in the content of the products degraded by caspase-1 or caspase-3 in response to an exposed dose of ionizing radiation, as shown in Fig. 4, is prepared. Then, the content of the products degraded by caspase-1 or caspase-3 in a tissue or blood collected from a living body is determined by Western blotting or the like, and the exposed dose of ionizing radiation is measured on the basis of the schematic illustration.
In a preferable embodiment of the present invention, there is a method wherein when there are a large number of samples to be used to measure the exposure dose of ionizing radiation, a primary screening is carried out by ELISA, and only samples suspected of being exposed to ionizing radiation are examined for their content of the products degraded by caspase-1 or caspase-3 by Western blotting thereby measuring the exposed dose of ionizing radiation.
In the present invention, the content of the products degraded by caspase-1 or caspase-3 in extracted proteins both when not exposed to ionizing radiation and after exposed to ionizing radiation are preferably determined. However, even when the content of the products degraded by caspase-1 or caspase-3 when not exposed to ionizing radiation cannot be determined, its sample can be suspected of being exposed to ionizing radiation by merely determining the content of the degraded products after exposure to ionizing radiation when a change in the content is significant.

The kit for measuring an exposed dose of ionizing radiation according to the present invention comprises an antibody for determining the content of at least one of the products of the LyGDI protein degraded by caspase-1 and caspase-3 by an immunoblotting method. The antibody is not particularly limited insofar as it is an antibody used in an immunoblotting method and raised against at least one of the products of the LyGDI protein degraded by caspase-1 and caspase-3.

The present invention will be described in more detail based on examples given below, but the invention is not meant to be limited by these.

### EXAMPLES

### Example 1 Test at the level of individual mice

### Example 1-1

### (Whole-body irradiation with X-ray)

Seven-week-old male mice (C57BU6NCrj (trade name), Japan Charles River) were raised in a usual environment to 8 to 9-week-old and subjected to whole-body irradiation with X-ray (ionizing radiation) at an exposure dose ratio of 0.6 Gy from an X-ray generator (Shin-ai Go (trade name), 200 kVp, 25 mA, manufactured by Shimadzu Corporation). The quantity of irradiation (exposed dose) was regulated by changing the irradiation time.

### (Collection of tissue and extraction of thymic protein)

After irradiation with the radiation, each mouse was dissected to collect thymus, and the collected tissue was minced by means of a surgical knife and solubilized with a SDS-sample buffer to prepare a solubilized sample. The SDS-sample buffer was composed of 5% glycerol, 25 mM Tris-HCl (pH 6.8), and 1% SDS.

### (Electrophoresis)

Was prepared a 12% polyacrylamide gel (resolving gel), and a 4% polyacrylamide gel (concentrating gel) was layered thereon, and the above solubilized sample was applied in an amount of 20 µg per lane and then electrophoresed (electrophoresis conditions were as follow: the migration current in the concentrating gel was 20 mA; the migration current in the resolving gel was 40 mA; the running buffer used was a Tris-glycine buffer). After the electrophoresis was finished, the concentrating gel was cut off, and the resolving gel was equilibrated with a transfer buffer for 5 minutes (twice).

In this connection, the resolving gel was prepared by mixing 3 mL of 40% acrylamide stock solution, 2.5 mL of Lower gel buffer (1.5 M Tris-HCl (pH 8.8), 0.4% SDS), 4.5 mL of sterilized milli Q water, 50 µL of 10% aqueous ammonium persulfate, and 10 µL of N,N,N',N'-tetramethyl ethylene diamine.

The concentrating gel was prepared by mixing 0.5 mL of 40% acrylamide stock solution, 1.25 mL of Upper gel buffer (0.5 M Tris-HCl (pH 6.8), 0.4% SDS), 3.25 mL of sterilized milli Q water, 30 µL of 10% aqueous ammonium persulfate, and 8 µL of N,N,N',N'-tetramethyl ethylene diamine.

The Tris-glycine buffer was composed of 25 mM Tris, 192 mM glycine and 0.1 % SDS, and the transfer buffer was composed of 25 mM Tris, 192 mM glycine and 10% methanol.

### (Immunoblotting)

The equilibrated gel was transferred onto a polyvinylidene difluoride membrane (PVDF manufactured by Nippon Eidoh Co., Ltd.) in a transfer buffer (100 mA, 3 hours) by semidry method. After transfer, the PVDF membrane was subjected to a blocking for 1 hour with PBST containing 5% skim milk.

After blocking was finished, an antibody recognizing the C-terminal of the LyGDI protein (Catalog No. Sc-604, manufactured by Santa Cruz) was bound thereto for 1 hour, and washings were carried out twice with PBST for 5 minutes. Then, an alkali phosphatase-labeled anti-mouse IgG (H+L) (manufactured by Promega) was bound to the membrane over for 1 hour, and then washings were carried out 3 times with PBST for 5 minutes. The membrane was rinsed with 0.1 M aqueous Tris solution (pH 9.5) for 5 minutes and then incubated for 5 minutes at room temperature with CDP-Star (registered trademark) Western Blot Chemiluminescence Reagent (manufactured by NEN Life Science Products, Inc.). After incubation, it was exposed onto a film (manufactured by Amersham Bioscience).

### (Results)

The results are shown in Fig. 3. As shown in Fig. 3(a), it was found that products degraded by caspase-1 (17 kDa) were not found in a low range of exposed dose (up to 0.1 Gy), but were found again at an exposed dose of 0.5 Gy or more. In addition, it was also found that a large amount of products degraded by caspase-3 (21 kDa) was found when the whole-body irradiation was 5 Gy or more. Further, as shown in Fig. 3(b), there was observed a decrease in the content of the products degraded by caspase-1 (17 kDa) even by irradiation with a low dose of 4 mGy.

From the above results, changes in the content of the products degraded by caspase-1 or caspase-3 against the exposed dose are schematically shown in Fig. 4. As is evident from these figures, there is a predetermined relationship between the exposed dose and the patterns of the products degraded by caspase-1 and caspase-3, and the content of these products degraded by caspase is quantified thereby enabling estimation of the quantity of ionizing radiation applied to the individual subject.

In this connection, the 21-kDa band on the film was confirmed to be derived from the products degraded by caspase-3 by immunoblotting of the same PVDF membrane with an antibody recognizing the caspase-3 cleavage site (an antibody recognizing the N-terminal side of the long chain after cleavage/KLH conjugates wherein the antibody was a monoclonal antibody raised against an oligopeptide consisting of the amino acid sequence SKLNYKPPPQKC (SEQ ID NO: 7) as antigen) (data are not shown). In addition, the N-terminal-side fragments of the LyGDI protein estimated to occur upon cleavage with the respective caspases were also confirmed by immunoblotting of the same PVDF membrane with an antibody (Catalog No. 66456E, manufactured by Phamingen) recognizing the N-terminal of the LyGDI protein (data not shown). On the basis of such information, it was confirmed that the 17-kDa band was derived from the products degraded by caspase-1 and the 21-kDa band was derived from the products degraded by caspase-3.

### Example 1-2

### (Measurement with bonemarrow)

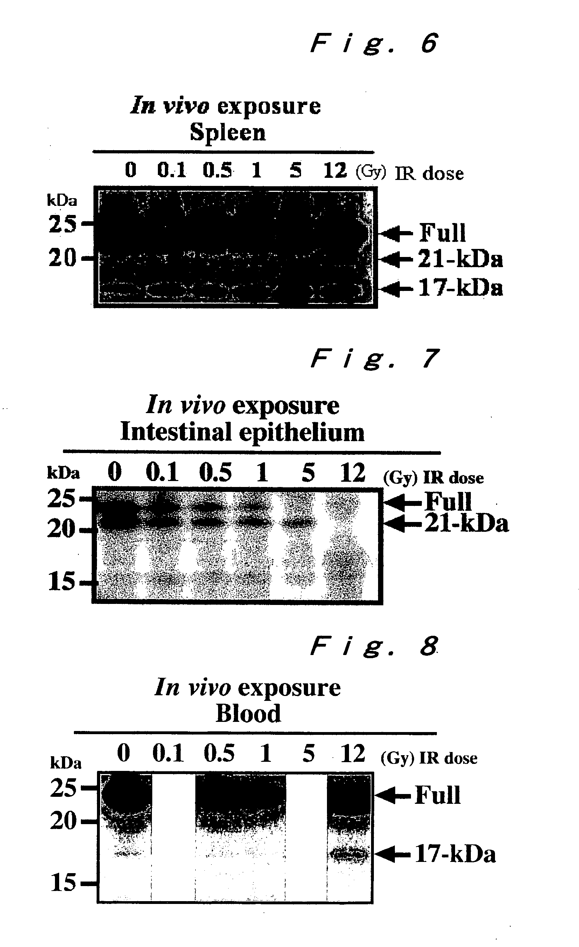

The content of the products degraded by caspase-1 and caspase-3 was examined by the same procedure as in Example 1-1, except that bonemarrow was used. The results are shown in Fig. 5. As shown in this figure, a very small amount of the products degraded by caspase-1 (17 kDa) constantly existed in an unirradiated subject, but the products were not found in a dose range of 1 Gy or more. In addition, in a dose range of 0.5 Gy or more, the amount of the full-length LyGDI protein tended to decrease, while a protein reacting specifically with the mouse antibody recognizing the C-terminal of the LyGDI protein, which was increased in reverse proportion to the decreasement of the full-length LyGDI protein, was observed in the vicinity of 13 kDA.

### Example 1-3

### (Measurement with spleen)

The content of the products degraded by caspase-1 and caspase-3 was examined by the same procedure as in Example 1-1, except that spleen was used. The results are shown in Fig. 6. As shown in this figure, the products degraded by caspase-1 (17 kDa) were observed constantly in the case of the spleen. Further, a very small amount of the products degraded by caspase-3 (21 kDa) was observed in a dose range of 1 Gy or more.

### Example 1-4

### (Measurement with intestinal epithelium tissue)

The content of the products degraded by caspase-1 and caspase-3 was examined by the same procedure as in Example 1-1, except that intestinal epithelium tissue was used. The results are shown in Fig. 7. As shown in this figure, the products degraded by caspase-3 (21 kDa) constantly existed in the unirradiated state (0 Gy) in the case of the intestinal epithelium tissue. On the other hand, the full-length LyGDI protein was not observed in a range of 5 Gy or more, and the products degraded by caspase-3 were not observed either in a range of 12 Gy or more. Further, the products degraded by caspase-1 hardly existed. This is possibly due to the drop out intestinal epithelium cells by irradiation with radiations.

### Example 1-5

### (Measurement with blood)

The content of the products degraded by caspase-1 and caspase-3 was examined by almost the same procedure as in Example 1-1, except that blood was used. The results are shown in Fig. 8. As shown in this figure, the products degraded by caspase-1 (17 kDa) were observed in a high dose range of 12 Gy or more. On the other hand, the products degraded by caspase-3 were hardly found.

From the results in Examples 1-1 to 1-5, it could be confirmed that the patterns of the products degraded by caspase-1 and caspase-3 in the tissues and blood collected from the mice changed depending on the exposed dose of ionizing radiation; that is, it could be confirmed that the exposed dose of ionizing radiation can be measured by detecting the patterns of the products degraded by caspase-1 and caspase-3.

### Example 2 Test at the human level

### Example 2-1

### (Irradiation with ionizing radiation)

A subject (adult man, 49 years old) was subjected in a usual manner once to dental panoramic radiography at the cervical part (radiation dose: about 20 mGy) with a dental panoramic radiographic device (trade name: auto 1000ex, manufactured by Asahi Roentgen Co., Ltd.). During photographing, the subject wore a lead apron.

### (Collection of blood and preparation of sample)

Before the radiation and 6 hours after the radiation, blood (peripheral blood) was collected, and white blood cells were separated therefrom and lyzed with an SDS-sample buffer to quantitate their protein amounts.

### (Electrophoresis and Western blotting)

The proteins were separated by SDS-PAGE and then subjected to Western blotting with the following 4 antibodies:
Antibody 1: sc-6047G (trade name, goat polyclonal antibody whose epitope was the C-terminal region of the LyGDI, manufactured by Santa Cruz)
Antibody 2: 66586E (trade name, rabbit polyclonal antibody raised against the full-length the LyGDI as antigen and not subjected to epitope mapping, manufactured by Pharmingen)
Antibody 3: 71-6300 (trade name, rabbit polyclonal antibody whose epitope was the central part of the LyGDI, manufactured by Zymed)
Antibody 4: 97A1015 (trade name, mouse monoclonal antibody recognizing the N-terminal of the Δ19-LyGDI, manufactured by Active Motif)

### (Results)

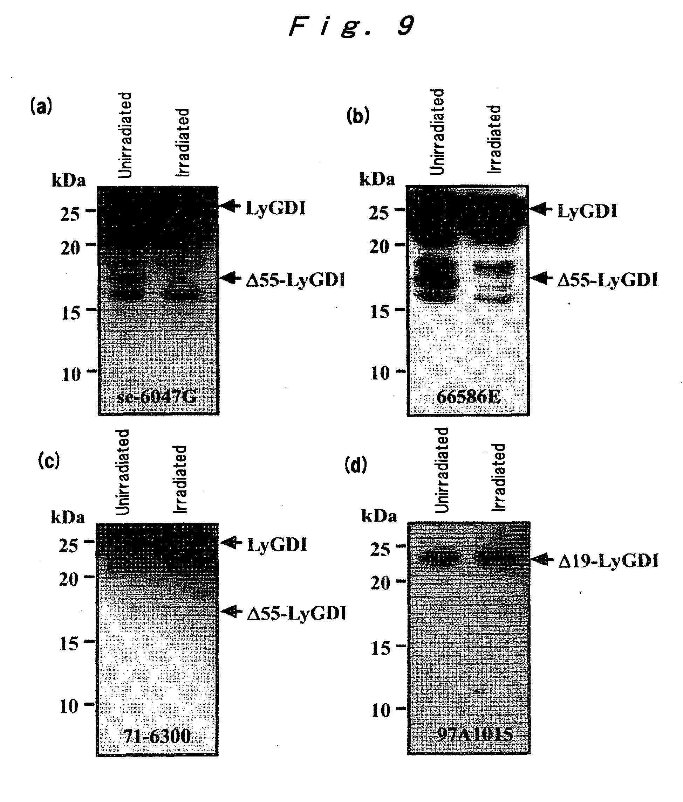

The results are shown in Fig. 9. As shown in Figs. 9(a), (b) and (c), the content of the Δ55-LyGDI decreased after irradiation than when unirradiated. On the other hand, the content of the Δ19-LyGDI when unirradiated was not different from that after irradiation.

### Example 2-2

### (Irradiation with ionizing radiation)

A subject (tumor bearing patient, 67 years old) was irradiated abdominally with ionizing radiations (radiation dose: about 2 Gy) by a liniac irradiation device for cancer therapy (trade name: Mebatron 67-6300, manufactured by Siemens AG).

### (Collection of blood and preparation of sample)

Before the radiography and 6 hours after the radiography, blood (peripheral blood) was collected, and white blood cells were separated therefrom and lyzed with a SDS-sample buffer, to quantitate for their protein amounts.

### (Electrophoresis and Western blotting)

The proteins were separated by SDS-PAGE and then subjected to Western blotting with the following 3 antibodies:
Antibody 1: sc-6047G (trade name, manufactured by Santa Cruz)
Antibody 2: 66586E (trade name, manufactured by Pharmingen)
Antibody 4: 97A1015 (trade name, manufactured by Active Motif)

### (Results)

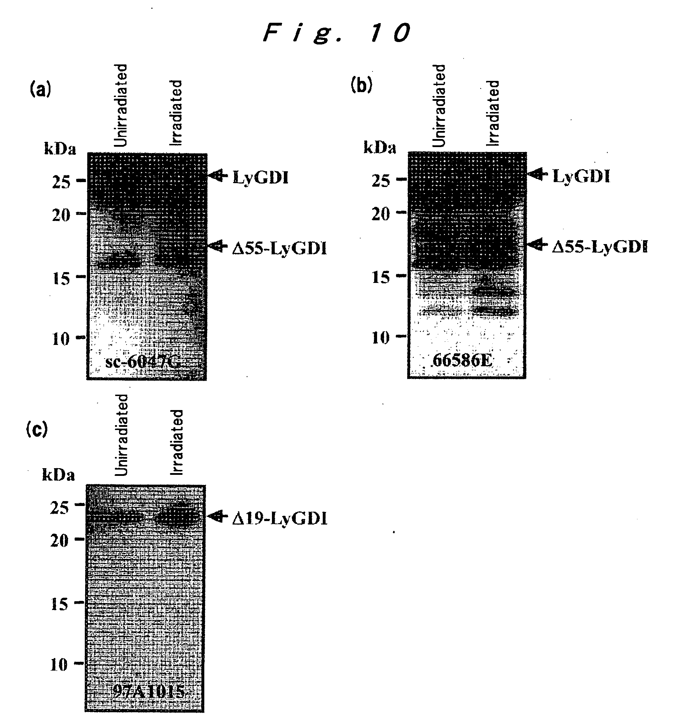

The results are shown in Fig. 10. As shown in Fig. 10(a), (b) and (c), the content of both the Δ55-LyGDI and the Δ19-LyGDI increased after the irradiation compared with the case of the unirradiated state.

In the foregoing results, it was revealed that in the case of irradiation with radiations in a low dose range such as in panoramic radiography, a decrease in the content of the Δ55-LyGDI was observed 6 hours after irradiation of ionizing radiations. In addition, the Δ19-LyGDI constantly existed in white blood cells in peripheral blood of human in the case of mouse, and its content did not change by panoramic radiography.
In the case of irradiation with radiations in a relatively high dose range such as in irradiation with radiations for cancer therapy, on the other hand, the content of the Δ19-LyGDI was increased with an increase in the content of the Δ55-LyGDI.
These results show that not only in mice but also in humans, changes in LyGDI-derived fragments such as the Δ55-LyGDI and the Δ19-LyGDI can be a biological marker indicative of exposure to ionizing radiation as well as for examining the exposed dose of ionizing radiation.

### INDUSTRIAL APPLICABILITY

The influence of ionizing radiation on a living body can be directly measured without previously having a measuring device, by the method of measuring an exposed dose of ionizing radiation according to the present invention. In addition, a film badge or the like is not necessary at the time of exposure, so even in the unlikely event that a living body is exposed to ionizing radiation, such as in an accident in nuclear fuel fabrication, in an accident in an atomic power plant or by accidental leakage of a nuclear material from a nuclear weapon or the like, the influence of the ionizing radiation on the living body can be known more accurately.

Having described our invention as related to the present embodiments, it is our intention that the invention not be limited by any of the details of the description, unless otherwise specified, but rather be construed broadly within its spirit and scope as set out in the accompanying claims.

## Claims

1. A method of measuring an exposed dose of ionizing radiation, comprising the steps of:
(a) extracting proteins from a tissue or blood collected from a living body, and
(b) determining the content of at least one of products of LyGDI protein degraded by caspase-1 and caspase-3 in the extracted proteins.

2. The method of measuring an exposed dose of ionizing radiation according to Claim 1, wherein the content of products of LyGDI protein degraded by caspase-1 and products of LyGDI protein degraded by caspase-3 are determined.

3. The method of measuring an exposed dose of ionizing radiation according to Claim 1 or 2, wherein the content of products of LyGDI protein degraded by caspase-1 and caspase-3 is determined by an immunoblotting method.

4. The method of measuring an exposed dose of ionizing radiation according to any one of Claims 1 to 3, wherein the protein is extracted from collected thymus, bonemarrow, spleen, intestinal epithelium tissue or blood.

5. The method of measuring an exposed dose of ionizing radiation according to any one of Claims 1 to 3, wherein the protein is extracted from collected thymus.

6. The method of measuring an exposed dose of ionizing radiation according to any one of Claims 1 to 3, wherein the protein is extracted from collected blood.

7. A kit for measuring an exposed dose of ionizing radiation, which comprises an antibody for determining, by an immunoblotting method, the content of at least one of products of LyGDI protein degraded by caspase-1 and caspase-3 in proteins extracted from a tissue or blood collected from a living body.
